Europäisches Patentamt

European Patent Office

Office européen des brevets

(19)

(11) **EP 0 636 357 B1**

(12)

# EUROPÄISCHE PATENTSCHRIFT

(45) Veröffentlichungstag und Bekanntmachung des Hinweises auf die Patenterteilung:
**12.05.1999 Patentblatt 1999/19**

(51) Int. Cl.$^6$: **A61K 7/06**, A61K 7/50, A61K 7/09

(21) Anmeldenummer: **94104919.9**

(22) Anmeldetag: **29.03.1994**

(54) **Haarbehandlungsmittel und Verfahren zur Anwendung**

Hair treatment agent and process for its use

Agent de soin capillaire et procédé d'utilisation

(84) Benannte Vertragsstaaten:
**DE ES FR GB IT**

(30) Priorität: **31.07.1993 DE 4325817**

(43) Veröffentlichungstag der Anmeldung:
**01.02.1995 Patentblatt 1995/05**

(73) Patentinhaber:
**Wella Aktiengesellschaft**
**64274 Darmstadt (DE)**

(72) Erfinder:
- **Kischka, Karl-Heinz, Dr.**
  **D-64293 Darmstadt (DE)**
- **Hoch, Dietrich**
  **D-64319 Pfungstadt-Eich (DE)**
- **Schmenger, Jürgen**
  **D-64331 Weiterstadt (DE)**
- **Flemming, Ernst**
  **D-63150 Heusenstamm (DE)**

(56) Entgegenhaltungen:
EP-A- 0 114 414      EP-A- 0 165 397
EP-A- 0 365 825      EP-A- 0 502 304
WO-A-91/15185        WO-A-94/02115

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Gegenstand der Erfindung ist ein Mittel zur Behandlung der Haare auf wäßriger oder wäßrig-alkoholischer Basis, welches eine Kombination von Polyvinylpyrrolidon und/oder Polyvinylpyrrolidon-Vinylacetat-Copolymer mit einem amphoteren Tensid, einem kationischen Tensid und einer organischen Säure enthält eine nicht-disperse Lösung ist und nicht ausgespült wird. sowie Verfahren zur Anwendung des Mittels.

Die Haare werden durch Einwirkungen verschiedener Art in ihren physikalischen, chemischen und morphologischen Eigenschaften negativ beeinflußt. So wird das Haar durch kosmetische Behandlungen, wie wiederholtes Bleichen, Dauerwellen und Färben, aber auch durch häufiges Waschen der Haare mit entfettenden Tensiden, durch Klimaeinflüsse wie Feuchte- und Temperaturunterschiede oder die intensive Einwirkung von Sonnenlicht sowie durch mechanische Behandlung wie Bürsten, Kämmen und Frottieren, insbesondere im Bereich der Haarspitzen, stark strapaziert und geschädigt, während das Haar am Haaransatz eine gesunde ungeschädigte Struktur besitzt. Die meist unterschiedliche Haarstruktur zwischen Haaransatz und den Haarspitzen stellt bei der Dauerwellbehandlung der Haare ein großes Problem dar. Wird das Dauerwellmittel in seiner Wellwirksamkeit den strapazierten und geschädigten Haarspitzen angepaßt, so erhält man eine nur ungenügende Dauerverformung des Haares am Haaransatz. Paßt man dagegen das Dauerwellmittel in seiner Wellwirksamkeit der gesunden, ungeschädigten Struktur des Haares am Haaransatz an, so kann das Haar an der strapazierten Haarspitze so sehr geschädigt werden, daß es zu einer stark eingeschränkten Lockigkeit bis hin zum Abbrechen der Haare kommen kann.

Das in Dokument WO-91/15185 beschriebene kosmetische Mittel ist ein Shampoo und im wesentlichen zusammengesetzt aus: a) einem Tensid, b) einem Polymergemisch bestehend aus hydrophilen und hydrophoben Monomeren oder Mischungen davon, und c) einem Lösungsmittel, welches auch notwendigerweise ausgespült werden muß.

Bei der Dauerwellbehandlung der Haare mit keratinreduzierenden Verformungsmitteln wird im allgemeinen so verfahren, daß man die zuvor gewaschenen und mit einem Handtuch abfrottierten Haare mit einem Teil des Verformungsmittels durchfeuchtet, sodann in Strähnen aufteilt, diese Strähnen einzeln auf Dauerwellwickler wickelt und anschließend mit dem restlichen Verformungsmittel befeuchtet. Nach Beendigung des Verformungsvorganges werden die Haare mit Wasser gespült, sodann oxidativ fixiert, anschließend die Wickler entfernt, die Haare erneut mit Wasser gespült und gegebenenfalls mit einem Kurpräparat nachbehandelt.

Diese Verfahrensweise besitzt jedoch einige Nachteile. So kann eine derartige Verfahrensweise zu Schädigungen an den Händen des die Verformungsbehandlung ausführenden Friseurs führen (zum Beispiel Allergien oder anderen Hauterkrankungen), da die Hände über die gesamte für das Wickeln erforderliche, etwa 20 Minuten betragende, Zeitspanne mit dem Verformungsmittel in Kontakt kommen. Außerdem ist das vorstehend beschriebene Verfahren nicht sehr haarschonend, da die durch das Vorfeuchten der Haare mit dem Verformungsmittel eintretende Haarerweichung beim Wickelvorgang sehr leicht zu einer Überdehnung des Haares und als Folge hiervon zu Haarbruch und Haarausfall führen kann.

Zudem sind die Haare nach einer Dauerwellverformungsbehandlung meist stark strapaziert, so daß eine Nachbehandlung mit einem Haarkurmittel erforderlich ist, um dem Haar wieder einen natürlichen Griff und Glanz zu verleihen.

Es bestand daher die Aufgabe, ein Mittel zur Behandlung der Haare zur Verfügung zu stellen, welches das Haar, insbesondere die Haarspitzen, während des Dauerwellvorganges schützt und so eine gleichmäßige Verformung der Haare ermöglicht. Zudem soll das Mittel gute glanzgebende Eigenschaften besitzen ohne das Haar zu belasten.

Es wurde nunmehr gefunden, daß durch ein Haarbehandlungsmittel auf wäßriger oder wäßrig-alkoholischer Basis, dadurch gekennzeichnet, daß es

| | |
|---|---|
| (A) 0,1 bis 10 | Gewichtsprozent Polyvinylpyrrolidon und/oder Polyvinylpyrrolidon-Vinylacetat-Copolymer, |
| (B) 0,1 bis 9 | Gewichtsprozent mindestens eines amphoteren Tensids, |
| (C) 0,1 bis 5 | Gewichtsprozent mindestens eines kationischen Tensids und |
| (D) 0,1 bis 2,0 | Gewichtsprozent mindestens einer organischen Säure |

enthält, eine nicht-disperse Lösung ist und nicht ausgespült wird. die gestellte Aufgabe in hervorragender Weise erfüllt wird.

Das erfindungsgemäße Mittel enthält die Komponente (A) bevorzugt in einer Menge von 0,1 bis 4 Gewichtsprozent. Geeignete Polyvinylpyrrolidone werden beispielsweise unter der Handelsbezeichnung Luviskol® K 90 von der Firma BASF, Ludwigshafen, BRD und unter der Handelsbezeichnung PVP/K® 90 von der Firma ISP, Surrey, Großbritannien vertrieben. Geeignete Polyvinylpyrrolidon-Vinylacetat-Copolymere werden beispielsweise unter der Handelsbezeichnung Luviskol®-VA von der Firma BASF, Ludwigshafen, BRD vertrieben. Von den als Komponente (A) des erfindungsgemäßen Mittels geeigneten polyvinylpyrrolidon-Vinylacetat-Copolymeren ist jenes bevorzugt, das ein Verhältnis der Polyvinylpyrrolidonmonomere zu den Vinylacetatmonomeren von 60 zu 40 aufweist und beispielsweise von der Firma BASF, Ludwigshafen, BRD unter der Handelsbezeichnung Luviskol® VA 64 vertrieben wird.

Das erfindungsgemäße Haarbehandlungsmittel enthält als Komponente (B) bevorzugt 0,1 bis 4 Gewichtsprozent mindestens eines amphoteren Tensids.

Beispiele für als Komponente (B) geeignete amphotere Tenside sind Alkylbetaine, Alkylaminobetaine, Fettsäureamidoalkylbetaine und Fettsäureamidoalkylsulfobetaine.

Das erfindungsgemäße Mittel enthält als Komponente (B) bevorzugt ein Fettsäureamidoalkylbetain und/oder ein Fettsäureamidoalkylsulfobetain.

Besonders bevorzugt enthält das erfindungsgemäße Mittel als Komponente (B) Kokosfettsäureamidopropyl-betain, das beispielsweise von der Firma Goldschmidt, Essen, BRD unter der Handelsbezeichnung Tego Betain® L7 vertrieben wird, und/oder 3-(3-Kokosfettsäureamidopropyl)dimethylammonium-2-hydroxypropan-sulfonat, das beispielsweise in Form einer 50-prozentigen wäßrigen Lösung unter der Handelsbezeichnung Rewoteric® AM-CAS von der Firma Rewo Chemische Werke GmbH, Steinau, BRD vertrieben wird.

Das erfindungsgemäße Mittel enthält bevorzugt 0,1 bis 2 Gewichtsprozent der Komponente (C).

Beispiel für als Komponente (C) des erfindungsgemäßen Mittels geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl-oder Cetylpyridiniumchlorid, Alkylamidethyltrimethylammoniumethersulfate sowie Verbindungen mit kationischen Charakter wie Aminoxide, beispielsweise Alkylmethylaminoxide oder Alkylaminoethyldimethylaminoxide.

Das erfindungsgemäß Mittel enthält als Komponente (C) bevorzugt Cetyltrimethylammoniumchlorid, das beispielsweise in Form einer 26-prozentigen wäßrigen Lösung unter der Handelsbezeichnung Dehyquart® A von der Firma Henkel KGaA, Düsseldorf, BRD und unter der Handelsbezeichnung Genamin® CTAC von der Firma Hoechst AG, Frankfurt, BRD sowie in Form einer 50-prozentigen Lösung in Isopropanol unter der Handelsbezeichnung Arquad® 16-50 von der Firma Akzo Chemicals GmbH, Düren, BRD vertrieben wird.

Das erfindungsgemäße Mittel enthält bevorzugt 0,1 bis 1 Gewichtsprozent der Komponente (D).

Das Mittel zur Behandlung der Haare gemäß der vorliegenden Erfindung enthält als Komponente (D) mindestens eine organische Säure, welche bevorzugt ausgewählt ist aus Weinsäure, Glyoxylsäure, Milchsäure und Ameisensäure.

In einer bevorzugten Ausführungsform enthält das erfindungsgemäße Mittel, zusätzlich zur Kombination der Komponenten (A), (B), (C) und (D), 0,01 bis 1 Gewichtsprozent, besonders bevorzugt 0,01 bis 0,1 Gewichtsprozent, eines Keratinhydrolysats, das beispielsweise in Form einer 20-prozentigen wäßrigen Lösung unter der Handelsbezeichnung Keratin Liquid® von der Firma Cosnaderm, Ladenburg, BRD und von der Firma Croda GmbH, Nettetal, BRD unter der Handelsbezeichnung Crotein® WKP vertrieben wird.

Das erfindungsgemäße Mittel weist bevorzugt einen pH-Wert von 2,5 bis 4,5 auf.

Das Haarbehandlungsmittel gemäß der vorliegenden Erfindung kann zusätzlich alle diejenigen Bestandteile enthalten, die in Haarbehandlungsmitteln üblicherweise eingesetzt werden, insbesondere Schaumsynergisten; Schaumstabilisatoren Sequestriermittel; Naturstoffe; Pigmente; parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; Puffersubstanzen, wie beispielsweise Natriumcitrat oder Natriumphosphat, in einer Menge von 0,1 bis 1,0 Gewichtsprozent; sowie Farbstoffe, wie zum Beispiel Fluorescein-Natriumsalz, Gelb ZN3 (C.I. 47 055), in einer Menge von 0,1 bis 1,0 Gewichtsprozent; weiterhin haarpflegende Zusätze, wie zum Beispiel Fettsäureester, Fettalkohole, Fettsäureglyceride, ethoxylierte oder propoxylierte gesättigte Fettalkohole; natürliche, modifizierte natürliche oder synthetische Polymere, wie zum Beispiel Schellack, kationische, anionische oder nichtionische Cellulosederivate, Chitosan, kationische Chitin- oder Chitosanderivate oder Polymerisate von Acrylsäurederivaten; Pflegestoffe, wie zum Beispiel Lanolinderivate, Cholesterin und pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; Antischuppenwirkstoffe; kosmetische Öle und Wachse sowie Konservierungsstoffe, soweit solche Zusätze nützlich und zweckmäßig erscheinen und mit den Bestandteilen des erfindungsgemäßen Mittels verträglich sind.

Das erfindungsgemäße Mittel weist bevorzugt einen Wassergehalt von 60 bis 98,5 Gewichtsprozent und kann Alkohol in einer Menge von 0,5 bis 20 Gewichtsprozent enthalten. Als Alkohol kommen hierbei insbesondere die für kosmetische Zwecke üblicherweise verwendeten niederen Alkohole mit 1 bis 4 Kohlenstoffatomen, wie zum Beispiel Ethanol und Isopropanol in Betracht.

Das erfindungsgemäße Haarbehandlungsmittel liegt in Form einer Zubereitung vor, die nach der Anwendung nicht ausgespült wird.

Das erfindungsgemäße Mittel kann als Haarstrukturregenerator vor der Wasserwelle, der Fönwelle, der Heißwelle oder vor einer Dauerwellbehandlung angewandt werden.

Das erfindungsgemäße Mittel weist hervorragende konditionierende Eigenschaften auf. Das erfindungsgemäße Haarbehandlungsmittel verleiht dem Haar einen schönen Glanz ohne die Haltbarkeit der Frisur zu beeinträchtigen. Die Kämmbarkeit und der Griff des Haares werden durch die Verwendung des erfindungsgemäßen Mittels deutlich verbessert.

Das erfindungsgemäße Mittel wird bevorzugt als Dauerwellvorbehandlungsmittel, das heißt zur Behandlung des Haares vor dem Wickeln auf Dauerwellwickler, verwendet.

Als Dauerwellvorbehandlungsmittel verwendet, bewirkt das erfindungsgemäße Mittel eine gleichmäßige Verformung der Haare während des Dauerwellvorgangs. Die empfindlichen Haarspitzen und strukturgeschädigtes Haar werden durch die Verwendung des erfindungsgemäßen Mittels als Dauerwellvorbehandlungsmittel während des Dauerwellvorganges geschützt, so daß eine schonende Dauerverformung des Haares ermöglicht wird.

Insbesondere strukturgeschädigtes Haar weist poröse Bereiche überwiegend in der Schuppenschicht, aber auch im Faserstamm, auf. Diese porösen Bereiche werden durch das erfindungsgemäße Mittel ausgeglichen, das bevorzugt die geschädigten Bereiche des Haares nachweisbar umhüllt, so daß die haarschädigende Wirkung der Dauerwellösung vermindert werden kann.

Zudem wird insbesondere durch die Komponente (D) des erfindungsgemäßen Mittels die Schließung der Schuppenschicht (Cuticula) des Haares gefördert, so daß die Diffusion der wellaktiven Bestandteile der Dauerwelllösung in das Haar verzögert wird.

Durch die Verwendung des erfindungsgemäßen Haarbehandlungsmittels als Dauerwellvorbehandlungsmittel wird zudem das Wickeln der Haare auf die Dauerwellwickler erheblich erleichtert. Darüberhinaus bewirkt das erfindungsgemäße Mittel, wenn es vor einer Dauerwellbehandlung angewendet wird, eine deutliche Verbesserung der Sprungkraft und der Elastizität des dauergewellten Haares.

Das erfindungsgemäße Mittel liegt vorzugsweise in Form einer wäßrigen oder wäßrig-alkoholischen Lösung vor und kann unter Verwendung eines Treibmittels oder mit Hilfe einer mechanisch betriebenen Sprühvorrichtung versprüht werden.

Wenn das erfindungsgemäße Mittel mit Hilfe eines Treibmittels versprüht wird, so enthält es bevorzugt 3 bis 75 Gewichtsprozent des Treibmittels und wird in einem Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Treibmittel geeignet.

Unter mechanischen Sprühvorrichtungen sind solche Vorrichtungen zu verstehen, welche das Versprühen einer Flüssigkeit ohne Verwendung eines Treibmittels ermöglichen. Als geeignete mechanische Sprühvorrichtung kann beispielsweise eine Sprühpumpe oder ein mit einem Sprühventil versehender elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Die vorliegende Erfindung betrifft daher weiterhin ein Verfahren zur dauerhaften Verformung von Haaren, bei dem man das Haar vor dem Wickeln auf Dauerwellwickler mit einem Dauerwellvorbehandlungsmittel behandelt, trocknet, erneut mit Wasser anfeuchtet, auf Wickler wickelt, sodann mit einem Haardauerverformungsmittel behandelt, nach einer für die dauerhafte Verformung des Haares ausreichenden Einwirkungszeit mit Wasser spült, sodann oxidativ nachbehandelt, mit Wasser spült, anschließend zur Frisur legt und sodann trocknet, welches dadurch gekennzeichnet ist, daß als Dauerwellvorbehandlungsmittel das vorstehend beschriebene erfindungsgemäße Haarbehandlungsmittel verwendet wird.

Bei dem erfindungsgemäßen Verfahren wird das Haar zunächst mit einem Shampoo gewaschen und danach mit Wasser gespült. Anschließend wird eine für die Dauerwellvorbehandlung ausreichende Menge, vorzugsweise etwa 10 bis 20 g, des erfindungsgemäßen Haarbehandlungsmittels auf das handtuchtrockene Haar, vorzugsweise auf die Haarspitzen, aufgetragen. Nach einer ausreichenden Einwirkungszeit, welche in Abhängigkeit von der Haarbeschaffenheit 5 bis 15 Minuten beträgt, wird das Haar getrocknet. Sodann wird das Haar mit Wasser angefeuchtet, ohne jedoch das Dauerwellvorbehandlungsmittel aus dem Haar auszuspülen. Das Haar wird in Strähnen aufgeteilt und auf Dauerwellwickler gewickelt. Der Durchmesser der Wickler beträgt hierbei etwa 5 bis 35 Millimeter. Anschließend wird das Haar mit einer für die dauerhafte Haarverformung ausreichenden Menge, vorzugsweise etwa 60 bis 90 g, eines Haardauerverformungsmittels behandelt.

Bei dem Haardauerverformungsmittel handelt es sich insbesondere um eine wäßrige, alkalisch (pH = 7 bis 10) eingestellte Zubereitung, welche eine keratinreduzierende Mercaptoverbindung, wie zum Beispiel Cystein, Cysteamin, N-Acetyl-L-Cystein, Mercaptocarbonsäuren, beispielsweise Thioglykolsäure oder Thiomilchsäure, oder Salze von Mercaptocarbonsäuren, wie zum Beispiel Ammonium- und Guanidinsalze der Thioglykolsäure oder Thiomilchsäure, in einer Konzentration von etwa 2 bis 12 Gewichtsprozent enthält.

Die erforderliche Alkalität wird hierbei durch die Zugabe von Ammoniak, organischen Aminen, Ammonium- und Alkalicarbonaten oder -hydrogencarbonaten eingestellt. Es kommt aber auch ein neutral oder sauer (pH = 4,5 bis 7) eingestelltes Haarverformungsmittel in Betracht, das im wäßrigen Medium einen wirksamen Gehalt an Sulfiten oder Mercaptocarbonsäureestern aufweist.

Im ersteren Fall werden vorzugsweise Natrium- oder Ammoniumsulfit oder das Salz der schwefligen Säure mit einem organischen Amin, wie zum Beispiel Monoethanolamin und Guanidin, in einer Konzentration von etwa 2 bis 12 Gewichtsprozent (berechnet als $SO_2$) verwendet. Im letzteren Fall kommen insbesondere Thioglykolsäuremonoglykolester oder -glycerinester in einer Konzentration von etwa 5 bis 50 Gewichtsprozent (entsprechend einem Gehalt an freier Thioglykolsäure von 2 bis 16 Gewichtsprozent) zur Anwendung.

Das Mittel zur dauerhaften Haarverformung kann auch ein Gemisch der vorstehend genannten keratinreduzierenden Verbindungen enthalten.

Nach einer für die dauerhafte Verformung des Haares ausreichenden Einwirkungszeit, welche, je nach Haarbeschaffenheit, dem pH-Wert und der Verformungswirksamkeit des Verformungsmittels sowie in Abhängigkeit von der Anwendungstemperatur, etwa 10 bis 30 Minuten beträgt, wird das Haar mit Wasser gespült und anschließend oxidativ nachbehandelt ("fixiert"). Das Nachbehandlungsmittel wird, je nach Haarfülle, in einer Menge von etwa 50 bis 100 g verwendet.

Für die oxidative Nachbehandlung kann jedes beliebige, bisher für eine derartige Behandlung verwendete Nachbehandlungsmittel verwendet werden. Beispiele für in einem derartigen Nachbehandlungsmittel verwendbare Oxidationsmittel sind Natrium- und Kaliumbromat, Natriumperborat, Harnstoffperoxid und Hydrogenperoxid.

Die Konzentration des Oxidationsmittels ist in Abhängigkeit von der Anwendungszeit (in der Regel etwa 5 bis 15 Minuten) und der Anwendungstemperatur unterschiedlich. Üblicherweise liegt das Oxidationsmittel in dem wäßrigen Nachbehandlungsmittel in einer Konzentration von etwa 0,5 bis 10 Gewichtsprozent vor.

Sowohl das Mittel zur dauerhaften Haarverformung als auch das Mittel zur oxidativen Nachbehandlung kann in Form einer wäßrigen Lösung oder Emulsion sowie in verdickter Form auf wäßriger Basis, insbesondere als Creme, Gel oder Paste, vorliegen. Es ist ebenfalls möglich, dieses Mittel unter Druck in Aerosoldosen abzufüllen und daraus als Schaum zu entnehmen.

Anschließend werden die Wickler entfernt. Falls erforderlich, kann das abgewickelte Haar nun nochmals oxidativ nachbehandelt werden. Sodann wird das Haar mit Wasser gespült, zur Frisur gelegt und schließlich getrocknet.

Das vorstehend beschriebene erfindungsgemäße Verfahren zur dauerhaften Haarverformung ermöglicht eine schonende und gleichmäßige Umformung vom Haaransatz bis zu den Haarspitzen, das Haar zeigt eine hervorragende Naß- und Trockkämmbarkeit, einen angenehmen Griff und einen ansprechenden Glanz im getrockneten Zustand, sowie eine lockere, sprunghafte und gleichmäßige Dauerwellung, insbesondere im Bereich der Haarspitzen.

Ein weiterer Gegenstand der vorliegenden Anmeldung ist ein, auch ohne nachfolgende Dauerwellbehandlung anzuwendendes, Verfahren zur Behandlung von Haaren, bei dem man nach der Haarwäsche das erfindungsgemäße Mittel, je nach Haarfülle, in einer Menge von 10 bis 20 g im handtuchtrocknen Haar verteilt, gegebenenfalls das Haar zur Frisur legt und sodann das Haar trocknet.

Die mit dem erfindungsgemäßen Mittel behandelten Haare zeigen eine hervorragende Naß- und Trockenkämmbarkeit, einen angenehmen Griff und einen ansprechenden Glanz im getrockneten Zustand.

Folgende Beispiele sollen den Gegenstand der Erfindung näher erläutern:

**Beispiele**

**Beispiel 1: Haarbehandlungsmittel**

| | |
|---|---|
| 0,600 g | Polyvinylpyrrolidon |
| 0,400 g | 3-(3-Kokosfettsäureamidopropyl)dimethylammonium-2-hydroxypropansulfonat |
| 0,250 g | Cetyltrimethylammoniumchlorid |
| 0,100 g | Keratinhydrolysat (Keratin Liquid® der Firma Cosnaderm/Ladenburg) |
| 0,100 g | Glyoxylsäure |
| 0,250 g | Isopropanol |
| 98,300 g | Wasser |
| 100,000 g | |

**Beispiel 2: Haarbehandlungsmittel**

| | |
|---|---|
| 2,000 g | Polyvinylpyrrolidon |
| 0,750 g | 3-(3-Kokosfettsäureamidopropyl)dimethylammonium-2-hydroxypropansulfonat |
| 0,104 g | Cetyltrimethylammoniumchlorid |
| 0,200 g | Zitronensäure |
| 96,946 g | Wasser |
| 100,000 g | |

**Beispiel 3: Haarbehandlungsmittel**

| | | |
|---|---|---|
| 3,200 g | Polyvinylpyrrolidon | |
| 1,950 g | 3-(3-Kokosfettsäureamidopropyl)-dimethylammonium-2-hydroxypropansulfonat | |
| 0,234 g | Cetyltrimethylammoniumchlorid | |
| 0,500 g | Kamillenblütenextrakt (Extrapon® Kamille Spezial 2/033021 der Firma Dragoco, Holzminden/BRD) | |
| 0,250 g | Zitronensäure | |
| 93,866 g | Wasser | |
| 100,000 g | | |

**Beispiel 4: Haarbehandlungsmittel**

| | | |
|---|---|---|
| 0,700 g | Polyvinylpyrrolidon | |
| 0,900 g | 3-(3-Kokosfettsäureamidopropyl)dimethylammonium-2-hydroxypropansulfonat | |
| 0,550 g | Cetyltrimethylammoniumchlorid | |
| 0,140 g | Keratinhydrolysat | |
| 0,100 g | Zitronensäure | |
| 0,001 g | Gelb ZN3 (C.I. 47005) | |
| 0,550 g | Isopropanol | |
| 97,059 g | Wasser | |
| 100,000 g | | |

**Beispiel 5: Haarbehandlungsmittel**

| | | |
|---|---|---|
| 0,700 g | Polyvinylpyrrolidon | |
| 1,050 g | 3-(3-Kokosfettsäureamidopropyl)dimethylammonium-2-hydroxypropan-sulfonat | |
| 0,600 g | Cetyltrimetylammoniumchlorid | |
| 0,450 g | Milchsäure | |
| 97,200 g | Wasser | |
| 100,000 g | | |

**Beispiel 6: Haarbehandlungsmittel**

| | | |
|---|---|---|
| 2,000 g | Polyvinylpyrrolidon-Vinylacetat-Copolymer | |
| 0,750 g | 3-(3-Kokosfettsäureamidopropyl)dimethylammonium-2-hydroxypropansulfonat | |
| 0,104 g | Cetyltrimethylammoniumchlorid | |
| 0,200 g | Zitronensäure | |
| 96,946 g | Wasser | |
| 100,000 g | | |

**Patentansprüche**

1. Mittel zur Behandlung der Haare auf wäßriger oder wäßrig-alkoholischer Basis, dadurch gekennzeichnet, daß es

(A) 0,1 bis 10    Gewichtsprozent Polyvinylpyrrolidon und/oder PolyvinylpyrrolidonVinylacetat-Copolymer,

(B) 0,1 bis 9    Gewichtsprozent mindestens eines amphoteren Tensids,

(C) 0,1 bis 5    Gewichtsprozent mindestens eines kationischen Tensids und

(D) 0,1 bis 2,0    Gewichtsprozent mindestens einer organischen Säure

enthält, eine nicht-disperse Lösung ist und nicht ausgespült wird.

2. Mittel nach Anspruch 1, dadurch gekennzeichnet, daß es 0,1 bis 4 Gewichtsprozent der Komponente (A) enthält.

3. Mittel nach einem der Ansprüche 1 oder 2, dadurch gekennzeichnet, daß es als Komponente (B) ein Fettsäureamidoalkylbetain und/oder ein Fettsäureamidoalkylsulfobetain enthält.

4. Mittel nach einem der Ansprüche 1 bis 3, dadurch gekennzeichnet, daß es 0,1 bis 4 Gewichtsprozent der Komponente (B) enthält.

5. Mittel nach einem der Ansprüche 1 bis 4, dadurch gekennzeichnet, daß es 0,1 bis 2 Gewichtsprozent der Komponente (C) enthält.

6. Mittel nach einem der Ansprüche 1 bis 5, dadurch gekennzeichnet, daß es als Komponente (C) Cetyltrimethylammoniumchlorid enthält.

7. Mittel nach einem der Ansprüche 1 bis 6, dadurch gekennzeichnet, daß es 0,1 bis 1 Gewichtsprozent der Komponente (D) enthält.

8. Mittel nach einem der Ansprüche 1 bis 7, dadurch gekennzeichnet, daß die organische Säure der Komponente (D) ausgewählt ist aus Weinsäure, Glyoxylsäure, Milchsäure und Ameisensäure.

9. Mittel nach einem der Ansprüche 1 bis 8, dadurch gekennzeichnet, daß es 0,01 bis 1 Gewichtsprozent eines Keratinhydrolysats enthält.

10. Verfahren zur dauerhaften Verformung von Haaren, bei dem man das Haar vor dem Wickeln auf Dauerwellwickler mit einem Dauerwellvorbehandlungsmittel behandelt, das Haar trocknet, erneut mit Wasser anfeuchtet, auf Wickler wickelt, sodann mit einem Haardauerverformungsmittel behandelt, nach einer für die dauerhafte Verformung des Haares ausreichenden Einwirkungszeit mit Wasser spült, sodann oxidativ nachbehandelt, mit Wasser spült, anschließend zur Frisur legt und sodann trocknet, dadurch gekennzeichnet, daß als Dauerwellvorbehandlungsmittel ein Mittel nach einem der Ansprüche 1 bis 9 verwendet wird.

11. Verfahren nach Anspruch 10, dadurch gekennzeichnet, daß man das Dauerwellvorbehandlungsmittel in einer Menge von 10 bis 20 g anwendet.

12. Verfahren zur Behandlung von Haaren, dadurch gekennzeichnet, daß man nach der Haarwäsche das Mittel nach einem der Ansprüche 1 bis 9 in einer Menge von 10 bis 20 g im handtuchtrockenen Haar verteilt und sodann das Haar trocknet.

## Claims

1. Agent for the treatment of hair, on an aqueous or aqueous-alcoholic basis, characterised in that it contains

   (A) 0.1 to 10     percent by weight polyvinylpyrrolidone and/or polyvinylpyrrolidone/vinyl acetate copolymer,

   (B) 0.1 to 9     percent by weight of at least one amphoteric surfactant,

   (C) 0.1 to 5     percent by weight of at least one cationic surfactant and

   (D) 0.1 to 2.0     percent by weight of at least one organic acid,

   and is a non-disperse solution and is not rinsed out.

2. Agent according to claim 1, characterised in that it contains from 0.1 to 4 percent by weight of component (A).

3. Agent according to either claim 1 or claim 2, characterised in that it contains as component (B) a fatty acid amidoalkyl betaine and/or a fatty acid amidoalkyl sulphobetaine.

4. Agent according to any one of claims 1 to 3, characterised in that it contains from 0.1 to 4 percent by weight of component (B).

5. Agent according to any one of claims 1 to 4, characterised in that it contains from 0.1 to 2 percent by weight of component (C).

6. Agent according to any one of claims 1 to 5, characterised in that it contains as component (C) cetyltrimethylammonium chloride.

7. Agent according to any one of claims 1 to 6, characterised in that it contains from 0.1 to 1 percent by weight of component (D).

8. Agent according to any one of claims 1 to 7, characterised in that the organic acid of component (D) is selected from tartaric acid, glyoxylic acid, lactic acid and formic acid.

9. Agent according to any one of claims 1 to 8, characterised in that it contains from 0.01 to 1 percent by weight of a keratin hydrolysate.

10. Process for the permanent shaping of hair, in which the hair, before being wound onto permanent waving curlers, is treated with a permanent waving pretreatment agent, the hair is dried, dampened with water again, wound onto curlers, then treated with a permanent hair shaping agent, rinsed with water after a period of action sufficient for the permanent shaping of the hair, then subjected to an oxidative after-treatment, rinsed with water, then arranged in a style and subsequently dried, characterised in that an agent according to any one of claims 1 to 9 is used as the permanent waving pretreatment agent.

11. Process according to claim 10, characterised in that the permanent waving pretreatment agent is applied in an amount of from 10 to 20 g.

12. Process for the treatment of hair, characterised in that, when the hair has been washed, the agent according to any one of claims 1 to 9 is distributed in the towel-dried hair in an amount of from 10 to 20 g and then the hair is dried.

## Revendications

1. Produit de traitement des cheveux, à base aqueuse ou alcoolo-aqueuse caractérisé par le fait qu'il contient :

   (A) 0,1 à 10 % en poids de polyvinylpyrrolidone et/ou d'un copolymère polyvinylpyrrolidone-acétate de vinyle
   (B) 0,1 à 9 % en poids d'au moins un agent tensio-actif amphotère
   (C) 0,1 à 5 % en poids d'au moins un agent tensio-actif cationique, et
   (D) 0,1 à 2,0 % en poids d'au moins un acide organique,

   qu'il est une solution non dispersée et qu'il n'est pas rincé.

2. Produit selon la revendication 1, caractérisé en qu'il contient 0,1 à 4 % en poids du composant (A).

3. Produit selon l'une des revendications 1 ou 2, caractérisé en ce qu'il contient à titre de composant (B) une amidoalkylbétaïne d'acide gras et/ou une amidoalkylsulfobétaïne d'acide gras.

4. Produit selon l'une des revendications 1 à 3, caractérisé en ce qu'il contient de 0,1 à 4 % en poids du composant (B).

5. Produit selon l'une des revendications 1 à 4, caractérisé en ce qu'il contient de 0,1 à 2 % en poids du composant (C).

6. Produit selon l'une des revendications 1 à 5, caractérisé en ce qu'il contient à titre de composant (C) un chlorure de cétyltriméthylammonium.

7. Produit selon l'une des revendications 1 à 6, caractérisé en ce qu'il contient de 0,1 à 1 % en poids du composant (D).

8. Produit selon l'une des revendications 1 à 7, caractérisé en ce que l'acide organique du composant (D) est choisi parmi l'acide tartrique, l'acide glycylique, l'acide lactique et l'acide formique.

9. Produit selon l'une des revendications 1 à 8, caractérisé en ce qu'il contient de 0,01 à 1 % en poids d'un hydrolysat de kératine.

10. Procédé de formation permanente des cheveux, dans lequel on traite les cheveux avant enroulement sur des rouleaux de permanente, avec un produit de pré-traitement de permanente, on sèche les cheveux, on réhumidifie avec de l'eau, on enroule sur des rouleaux, puis l'on traite avec un produit de formage de permanente, après un temps d'action suffisant pour obtenir un formage permanent des cheveux, on rince à l'eau, puis l'on post-traite, de façon oxydative, on rince à l'eau, puis l'on met la coiffure en forme et ensuite l'on sèche, caractérisé en ce que l'on utilise comme produit de pré-traitement de permanente un produit selon l'une des revendications 1 à 9.

11. Procédé selon la revendication 10, caractérisé en ce que l'on utilise le produit de pré-traitement de permanente en une quantité allant de 10 à 20 g.

12. Procédé de traitement des cheveux, caractérisé en que, après avoir lavé les cheveux, on répartit le produit selon l'une des revendications 1 à 9 en une quantité de 10 à 20 g dans les cheveux séchés à la serviette, puis l'on sèche les cheveux.